# FASCICULE DE BREVET EUROPEEN

(11) **EP 3 132 048 B1**
(45) Date de publication et mention de la délivrance du brevet: **04.12.2019**
(21) Numéro de dépôt: 15725739.5
(22) Date de dépôt: 15.04.2015
(51) Int. Cl.: C12Q 1/6806, C12P 19/34

(54) **PROCÉDÉ DE SYNTHÈSE D'ACIDES NUCLÉIQUES, NOTAMMENT D'ACIDES NUCLÉIQUES DE GRANDE LONGUEUR, UTILISATION DU PROCÉDÉ ET KIT POUR LA MISE EN OEUVRE DU PROCÉDÉ**
VERFAHREN ZUR SYNTHESE VON NUKLEINSÄUREN, INSBESONDERE VON LANGEN NUKLEINSÄUREN, VERWENDUNG DIESES VERFAHRENS UND KIT ZUR IMPLEMENTIERUNG DIESES VERFAHRENS
METHOD FOR SYNTHESISING NUCLEIC ACIDS, IN PARTICULAR LONG NUCLEIC ACIDS, USE OF SAID METHOD AND KIT FOR IMPLEMENTING SAID METHOD

(30) Priorité: 17.04.2014 FR 1453455
(43) Date de publication de la demande: 22.02.2017
(60) Demande divisionnaire: 19195271.2
(73) Titulaire: DNA Script, 75005 Paris (FR)
(72) Inventeur: YBERT, Thomas, 75012 Paris (FR); GARIEL, Sylvain, 75003 Paris (FR)
(74) Mandataire: Cabinet Becker et Associés
(86) Numéro de dépôt international: PCT/FR2015/051022
(87) Numéro de publication internationale: WO 2015/159023

(56) Documents cités:
- WO-A1-00/60072
- WO-A2-2005/059096
- US-A- 5 808 045
- ANTHONY J. BERDIS ET AL: "The Use of Non-natural Nucleotides to Probe Template-Independent DNA Synthesis", CHEMBIOCHEM, vol. 8, no. 12, 13 août 2007 (2007-08-13), pages 1399-1408, XP055162789, ISSN: 1439-4227, DOI: 10.1002/cbic.200700096

### SEQUENCE LISTING

<110> DNA SCRIPT
<120> Procédé de synthèse d'acides nucléiques, notamment d'acides nucléiques de grande longueur, utilisation du procédé et kit pour la mise enoeuvre du procédé
<130> PP000434
<150> FR1453455
   <151> 2014-04-17
<160> 2
<170> PatentIn version 3.5
<210> 1
   <211> 20
   <212> DNA
   <213> acide nucléique
<400> 1
   gggggggggg ggggctgcag 20
<210> 2
   <211> 20
   <212> DNA
   <213> acide nucléique
<400> 2
   cccccccccc ccccgacgtc 20

## Description

La présente invention concerne un procédé de synthèse d'acides nucléiques, notamment d'acides nucléiques de grande longueur ainsi qu'un kit pour la mise en œuvre de ce procédé de synthèse.

### État de la technique

Les polymères d'acides nucléiques sont actuellement synthétisés *in vitro* par des méthodes de synthèse organique. La plus courante de ces méthodes est la méthode dite des phosphoramidites décrite par Adams et al. (1983, J. Amer. Chem. Soc. 105:661) et Froehler et al. (1983, Tetrahedron Lett. 24:3171).

Cette méthode de synthèse est très largement répandue et reste la plus couramment utilisée par les différents laboratoires et entreprises ayant des activités de production d'acides nucléiques. Par son degré d'utilisation et par les performances qui lui sont propres cette méthode constitue la référence actuelle en termes de synthèse d'acides nucléiques.

Cette méthode met en œuvre une réaction de couplage entre le dernier nucléotide d'un fragment d'acide nucléique et le nucléotide à ajouter. La première réaction de couplage étant réalisée entre un support solide et le premier nucléotide. Le couplage s'effectue entre le groupe 5'-OH du dernier nucléotide de l'acide nucléique et le groupe 3'-OH du nucléotide à ajouter. De cette façon la synthèse de l'acide nucléique est dite de type 3' vers 5'. Lors de la réaction de couplage, le groupement phosphoramidite est impliqué dans la réaction.

Chaque nucléotide à ajouter est protégé au niveau du groupe 5'-OH de façon à éviter une polymérisation incontrôlée de plusieurs nucléotides du même type, l'étape de couplage devant conduire à l'addition d'un seul nucléotide. Généralement la protection du groupe 5'-OH est réalisée par un groupement trityle. Afin d'éviter une éventuelle dégradation due à l'utilisation de réactifs puissants, les bases portées par les nucléotides peuvent également être protégées. Généralement la protection utilisée fait intervenir un groupement isobutyryle (Reddy et al. 1997, Nucleosides & Nucleotides 16:1589). Après chaque incorporation de nouveaux nucléotides le groupe 5'-OH du dernier nucléotide de la chaîne subit une réaction de déprotection de façon à le rendre disponible pour la prochaine étape de polymérisation. Les bases portées par les nucléotides composant l'acide nucléique, elles, ne sont déprotégées qu'après achèvement de la polymérisation.

Entre chaque étape d'ajout de nucléotide, une étape spécifique de neutralisation est réalisée. Elle consiste à modifier de façon permanente les groupes 5'-OH déprotégés des fragments n'ayant pas intégré de nucléotide à l'étape de couplage et dont la séquence est de fait incorrecte. Cette étape implique généralement une réaction d'acétylation.

Finalement une ultime étape d'oxydation (par exemple par traitement à l'iode) est nécessaire pour régénérer la liaison phosphodiester naturellement présente entre les nucléotides de molécules d'acides nucléiques.

Les méthodes de synthèse organique d'acides nucléiques, telle que celle exposée ci-dessus, nécessitent des quantités importantes de réactifs instables, dangereux, coûteux et pouvant avoir un impact sur l'environnement et la santé. Les différentes étapes mises en oeuvre sont également coûteuses et parfois difficiles à maîtriser. Enfin les dispositifs permettant de réaliser de façon pratique ces synthèses sont complexes, nécessitent un investissement important et doivent être opérés par une main d'oeuvre qualifiée et dédiée.

Un des désavantages majeur de ces techniques de synthèse organique réside dans leur faible rendement. Lors de chaque cycle la réaction de couplage n'intervient que dans 98 à 99,5% des cas, laissant dans le milieu réactionnel des acides nucléiques n'ayant pas une séquence correcte. Au fur et à mesure de la synthèse le milieu réactionnel s'enrichit donc fortement en fragments comportant une séquence totalement incorrecte. Les erreurs de type délétion qui surviennent ainsi ont des répercutions particulièrement dramatiques entraînant un décalage du cadre de lecture des fragments d'acides nucléiques considérés.

Ainsi pour une réaction de couplage correcte dans 99% des cas, un acide nucléique comportant 70 nucléotides sera synthétisé avec un rendement inférieur à 50%. Ce qui signifie qu'après 70 cycles d'addition, le milieu réactionnel comportera plus de fragments ayant une séquence erronée que de fragments ayant une séquence correcte. Ce mélange se révèle alors impropre à une utilisation ultérieure.

De manière générale, la proportion *Z* de fragments corrects contenus dans le mélange après *i* cycles d'addition de nucléotides, chaque addition ayant un taux de réussite *ρ* peut s'écrire : Z = *ρⁱ*

S'ajoutent aux problèmes entraînés par la limite de l'efficacité de couplage des problèmes identiques pour toutes les autres réactions impliquées dans le processus de synthèse. En effet, ces réactions ne sont pas toujours complètes et favorisent également la création de fragments d'acides nucléiques comportant une séquence erronée ou une structure moléculaire non conforme. Ces fragments viennent grossir les rangs des impuretés au fur et à mesure de l'enchainement des cycles de synthèse.

Les méthodes de synthèse organique d'acide nucléique se révèlent donc inefficaces pour la synthèse de fragments longs car elles génèrent une très grande quantité de fragments possédant une séquence incorrecte, alors considérés comme des impuretés. Dans la pratique, la longueur maximale des fragments pouvant être efficacement produits par ces méthodes est comprise entre 50 et 100 nucléotides.

Il existe des techniques alternatives utilisant un catalyseur enzymatique pour la réalisation de l'étape de couplage, notamment d'enzymes pouvant réaliser la réaction de couplage entre nucléotides en l'absence de brin matrice. Plusieurs enzymes de type polymérase semblent adaptées à ce genre ces méthodes de synthèse. Les enzymes à la catégorie des polymérases, ou des ligases, capables de créer une liaison phosphodiester entre deux nucléotides, plus particulièrement les enzymes de la famille des télomèrases, de la famille des enzymes de translésion de type polymérase η ou ζ, de la famille des PNPases, de la famille des RNA-polymérase template-independant, de la famille des terminal transférases ou de la famille des ligases font partie des enzymes pouvant jouer le rôle de catalyseur enzymatique. L'enzyme RNA ligase est utilisable dans ce cadre, ainsi que la terminal déoxynucléotidyl transférase (TdT).

Ces méthodes de synthèses dites enzymatiques permettent de se passer des solvants et réactifs instables, dangereux et coûteux utilisés lors des méthodes de synthèse organique. Les méthodes de synthèse enzymatiques effectuent une polymérisation des acides nucléiques dans le sens 5' vers 3'. Ainsi, Le couplage s'effectue entre le groupe 3'-OH du dernier nucléotide et le groupe 5'-OH du nucléotide à ajouter.

Dans certaines méthodes enzymatiques, l'enzyme permettant la polymérisation est directement ajoutée à des nucléotides naturels (Deng et al. 1983, Meth. Enzymol. 100 :96). A partir d'un fragment d'acide nucléique initial appelé *amorce,* l'enzyme de polymérisation ainsi que les nucléotides d'un même type sont ajoutés. La réaction de polymérisation est alors amorcée jusqu'à ce qu'elle soit arrêtée par une méthode physique ou chimique. L'acide nucléique grandit de façon séquentielle par répétition de ces étapes de création de liaison phosphodiester.

L'utilisation de nucléotides naturels entraine un phénomène de polymérisation incontrôlé aboutissant à un mélange très hétérogène de molécules d'acides nucléiques. En effet rien ne prévient l'addition de plusieurs nucléotides d'un même type après une première addition. Dans la pratique une telle méthode de synthèse se révèle inutilisable pour la synthèse de fragments d'acide nucléiques ayant une séquence désirée.

L'utilisation de nucléotides protégés permet dans une certaine mesure de résoudre ce phénomène de polymérisation incontrôlé, en empêchant de manière totale ou partielle la création de liaisons phosphodiester supplémentaires par rapport à celle désirée, et provoquant ainsi l'arrêt de la synthèse.

Un problème rencontré dans ce cas est celui de la synthèse de nucléotides protégés. La mise en place de la protection qui peut être localisée sur la base ou sur le groupe 3'-OH fait intervenir une succession de réactions chimiques complexes. A l'issue de la synthèse les nucléotides ayant la protection désirée sont rarement présents sous forme pure. Certaines impuretés telles que les nucléotides de départ, non protégés, affectent très négativement les rendements de synthèse.

Un autre problème rencontré concerne l'efficacité de protection des nucléotides. Suivant la stratégie retenue, l'efficacité de protection est parfois insuffisante pour garantir de bons rendements de synthèse. Dans ce cas, une stratégie efficace semble être la prévention totale de création d'une liaison covalente entre le nucléotide terminal et un nucléotide présent. Celle-ci se traduit généralement par la transformation du groupe 3'-OH en une autre fonction chimique incapable de subir une transformation de la part de l'enzyme.

Une autre difficulté concerne la capacité à déprotéger les nucléotides, une fois l'ajout effectué, de manière satisfaisante et avant l'ajout prévu pour le cycle suivant. Chaque fragment d'acide nucléique dont le dernier nucléotide échoue à être déprotégé ne pourra pas accepter le nucléotide suivant lors du prochain cycle. Ce fragment aura dès lors une séquence erronée ayant pour conséquence de faire baisser le rendement de la synthèse.

Un problème majeur rencontré par les méthodes de synthèse enzymatique est l'efficacité de la réaction de couplage. Bien qu'étant supérieure à celle des méthodes de synthèse organique, l'efficacité globale de couplage des méthodes enzymatiques est bien inférieure à 100%. Ainsi, des fragments ayant échoué à l'ajout d'un nucléotide supplémentaire subsistent dans le réactionnel.

En effet, l'enzyme doit pouvoir rencontrer ses substrats, un fragment d'acide nucléique d'une part et un nucléotide d'autre part, afin de réaliser la réaction de liaison. L'enzyme et le fragment d'acide nucléique étant des macromolécules leur rencontre peut parfois ne pas avoir lieu dans le temps imparti. Une concentration élevée d'enzyme et de fragments d'acide nucléique permet de minimiser ce phénomène sans toutefois l'éliminer complètement. A l'opposé, des méthodes mettant en jeux des ajouts successifs d'enzyme sans étape de lavage ou d'élimination des réactifs en excès, favorisent une trop grande dilution des réactifs aboutissant à des efficacités de couplage faibles.

Souvent, l'apparition de fragments possédant une séquence erronée, concourant à la baisse du rendement de synthèse, entraine l'ajout d'une étape de neutralisation. Cette étape consiste à éliminer les groupes 3'-OH n'ayant pas formé de liaison phosphodiester. Cette étape peut être réalisée par réaction chimique ou par l'action d'une enzyme, mais elle n'est, dans tous les cas, d'une part, pas efficace à 100%, et d'autre part, elle n'élimine pas les fragments concernés qui contribuent ainsi à l'hétérogénéité du mélange.

Dans certains procédés, après réaction, les enzymes et réactifs introduits sont inactivés par l'ajout d'autres enzymes ou réactifs ou par l'application de conditions physiques particulières. Cette stratégie permet d'éviter les étapes de lavage, mais implique une augmentation inexorable du volume réactionnel avec pour conséquence une dilution des réactifs et donc une baisse de l'efficacité et de la rapidité des réactions mises en jeu comme décrit ci-dessus. En outre, cette stratégie ne permet pas le recyclage de certains réactifs coûteux tels que l'enzyme de polymérisation par exemple. Enfin, cette stratégie engendre un nombre de déchets réactionnels conséquents issus de l'inactivation des réactifs initiaux. Ces déchets contribuent eux aussi à l'hétérogénéité du mélange final ainsi qu'à la baisse du rendement de synthèse par d'éventuelles inhibitions d'enzymes ou de réactifs.

Quelques soient les méthodes employées, un grand nombre d'impuretés s'accumulent au cours des cycles de synthèse. Les impuretés sont majoritairement constituées de fragments n'ayant pas la séquence de nucléotides attendue. Plusieurs stratégies d'élimination des fragments de séquences erronées sont disponibles, telles la chromatographie d'exclusion stérique ou électrophorèse sur gel polymère ou la fixation sur phase solide. Cependant, la première présente des performances si faibles qu'elle n'est pas envisageable pour la synthèse de fragments de plus d'une dizaine de nucléotides, la seconde suppose que le fragment d'acide nucléique initial soit fixé à un support solide non soluble. Ainsi à chaque cycle, des étapes simples de lavage avec retenue du support solide permettent l'élimination de toutes les impuretés, exceptés les fragments d'acide nucléique fixés. Bien qu'extrêmement performante, cette stratégie ne permet pas l'élimination des fragments n'ayant pas subi d'addition de nucléotides lors d'un cycle, ni ceux ayant été neutralisés. Ainsi les impuretés majoritaires ne sont pas éliminées ce qui contribue à la baisse significative du rendement de synthèse.

Par ailleurs, le document WO 2005/059096 décrit un procédé de synthèse d'acides nucléiques sur un support solide suivi de la détection et de la correction des séquences erronées. Ce procédé nécessite des étapes d'identification ou d'analyse complexes.

A ce jour aucun procédé de synthèse d'acide nucléique n'est réellement satisfaisant, et ne permet de répondre à la problématique de synthèse de fragments longs quelle que soit leur séquence. L'une des raisons premières est la perte de rendement systématique inhérente à toutes les méthodes proposées à ce jour. Notamment, cette perte de rendement est dépendante de la longueur de l'acide nucléique désiré et atteint des proportions telles qu'elle empêche la synthèse de longs fragments. Spécifiquement, la synthèse directe de fragments d'acide nucléique de la taille typique d'un gène, soit entre 500 et 5 000 nucléotides est totalement inaccessible par les procédés actuels.

Un premier but de l'invention est de pallier les inconvénients des méthodes de l'Art Antérieur en proposant un procédé de synthèse d'acides nucléiques avec un rendement élevé.

Un autre but de l'invention est de proposer un procédé de synthèse d'acides nucléiques de grande longueur, c'est-à-dire d'au moins plusieurs centaines ou plusieurs milliers de nucléotides.

Un autre but de l'invention est de proposer un procédé de synthèse comprenant une succession d'étapes qui combinées entre-elles permettent de conserver un rendement de synthèse extrêmement élevé et cela de façon indépendante de la taille du fragment d'acide nucléique à synthétiser.

### Exposé de l'invention

Ces buts sont atteints par le procédé de synthèse d'acides nucléiques selon la présente invention, définie par les revendications ci-jointes notamment d'acides nucléiques de grande longueur, comprenant au moins un cycle d'élongation de fragments d'acides nucléiques, dénommés fragments initiaux, comprenant n (ou de séquence à n) nucléotides, n allant de 3 à 10¹², chaque cycle se subdivisant de la manière suivante :
a)une phase d'addition enzymatique de Xi nucléotides à une extrémité desdits fragments, X pouvant être compris entre 1 et 5, de préférence entre 1 et 3, i étant le numéro du cycle, permettant d'obtenir des fragments comprenant n + Xi nucléotides, dénommée première phase, et comprenant les étapes suivantes :
   - une première étape d'accrochage, sur un premier support, de l'extrémité 5' des fragments d'acides nucléiques initiaux ou en cours d'élongation, renfermant n nucléotides, ledit premier support présentant une surface d'accrochage permettant une liaison réversible avec l'extrémité 5' desdits fragments,
   - une étape d'ajout des réactifs nécessaires à l'addition enzymatique, une étape d'addition enzymatique de Xi nucléotides à la seconde extrémité desdits fragments d'acides nucléiques, X pouvant être compris entre 1 et 5, de préférence entre 1 et 3, i étant le numéro du cycle, la dite extrémité libre du ou des nucléotides additionnés auxdits fragments comporte un groupement chimique protecteur, pour empêcher une addition multiple des Xi nucléotides sur un même fragment.
   - une étape de décrochage dudit premier support desdits fragments à n + Xi nucléotides,
   - une première étape de transfert desdits fragments à n + Xi nucléotides; par création d'un flux mobile de fluide vers un second support apte à interagir avec le groupement protecteur de l'extrémité 3' des fragments à n + Xi nucléotides au moyen d'une liaison non covalente,
b)une phase de purification des fragments ayant une séquence correcte comprenant n + Xi nucléotides, dénommée deuxième phase, comprenant les étapes successives suivantes :
   - une seconde étape d'accrochage, sur un second support, desdits fragments à n + Xi nucléotides par leur extrémité par leur extrémité 3',
   - une étape d'élimination des fragments non-additionnés et des fragments non-accrochés sur le second support,
   - une étape de décrochage desdits fragments à n + Xi nucléotides dudit second support
   - une étape éventuelle d'élimination du milieu réactionnel des réactifs résiduels indésirables;
c) une phase éventuelle d'amplification, de préférence enzymatique, telle que par PCR, des fragments ayant une séquence correcte à n+Xi nucléotides dénommée troisième phase, comprenant les étapes successives suivantes:
   - une étape d'ajout des réactifs nécessaires à l'amplification,
   - une étape (composée éventuellement de sous-étapes permettant le processus) de multiplication d'un facteur multiplicateur Yi des fragments à n + Xi nucléotides, i étant le numéro de cycle, Y pouvant être compris entre 1 et 4.10¹⁰, de préférence entre 1 et 1.10⁹,
   - une étape de transfert des fragments à n + Xi nucléotides,
chaque cycle étant réalisé dans un milieu réactionnel compatible avec une addition et une élongation enzymatiques, tel qu'un milieu aqueux, le procédé de synthèse comportant également à la fin de l'ensemble des i cycles d'élongation, une étape d'amplification finale d'un facteur multiplicateur Yf.

Chaque phase d'addition par catalyse enzymatique est ainsi suivie d'une phase de purification qui permet de ne conserver dans le milieu réactionnel que les fragments de séquence correcte comprenant n + Xi nucléotides., et ce sont ces seuls fragments qui seront soumis à la phase d'amplification éventuelle, soit entre chaque cycle, soit après un nombre déterminé de cycles, soit encore uniquement à la fin des i cycles d'élongation, lorsque les fragments d'acides nucléiques présentent la longueur souhaitée.

Le procédé de la présente invention permet ainsi de synthétiser des fragments d'acides nucléiques ayant la séquence désirée. Il s'affranchit de l'utilisation de fragments d'acides nucléiques modèles et permet la synthèse de produits d'une très haute pureté, indépendamment de la longueur et de la nature de la séquence choisie.

Plus particulièrement, la phase de purification des fragments de séquence correcte à n + Xi nucléotides, comprend l'élimination des fragments non-additionnés et/ou de tout autre réactif résiduel indésirable.

La phase d'amplification est notamment une amplification sélective des fragments de séquence à n + Xi nucléotides, dénommés fragments de séquence correcte, multipliant dans le milieu réactionnel les fragments ayant une séquence correcte par un facteur multiplicatif plus de 10 fois supérieur, de préférence plus de 100 fois supérieur, au facteur multiplicatif des autres fragments, dénommés fragments de séquence incorrect, notamment les fragments non-additionnés.

De préférence le premier support présente une surface sur laquelle sont fixés, de manière non covalente, des brins d'acides nucléiques à au moins trois nucléotides, lesdits nucléotides de ces brins étant complémentaires des nucléotides présents à la première extrémité des fragments initiaux, de manière à immobiliser par des liaisons hydrogène entre leurs bases respectives, les dites premières extrémités des fragments.

Lesdits premier et second supports sont avantageusement deux supports distincts se présentant, par exemple, sous la forme de plaque de verre, de plaque en matériau polymère, ou de billes. De préférence l'un ou l'autre de ces supports, avantageusement le second support, présente des propriétés magnétiques.

Le premier support et le second support présentent avantageusement des surfaces d'accrochage de natures différentes, lesdits premier et second supports étant de préférence distincts, notamment distincts dans leur localisation spatiale. L'un des supports solide peut être fixe, et l'autre par exemple mobile dans le milieu réactionnel ou apte à être déplacé.

Le procédé de synthèse selon la présente invention est basé sur l'utilisation de catalyseur enzymatique pour réaliser la liaison phosphodiester permettant la polymérisation des nucléotides composant les fragments d'acides nucléiques à réaliser. Plus spécifiquement, le procédé met en oeuvre, sans s'y limiter, des enzymes permettant la création de la liaison phosphodiester entre le groupe 3'-OH du fragment d'acide nucléique en cours de synthèse et le groupe 5'-OH du nucléotide à ajouter lors de l'étape d'addition enzymatique.

Dans un mode de réalisation préféré, les enzymes utilisées sont capables d'assurer la polymérisation des nucléotides indépendamment de la présence d'un modèle matriciel. De telles enzymes sont alors capables de synthétiser des acides nucléiques en l'absence d'un quelconque brin complémentaire dans le milieu. De plus ces enzymes ont la capacité de synthétiser des fragments d'acide nucléique simple brin.

L'addition de X nucléotides est donc avantageusement effectuée par voie enzymatique, au moyen d'enzymes aptes à polymériser des nucléotides modifiés sans présence de brin matrice.

L'enzyme est par exemple choisie parmi les enzymes de la famille des télomèrases, de la famille des enzymes de translésion, de type polymérase η ou ζ, de la famille des PNPases, de la famille des RNA-polymérase template-indépendant, de la famille des terminal transférases ou de la famille des ligases, les ADN-polymérases template-indépendant ou encore les enzymes de la famille des terminal déoxynucléotidyl transférases (TdT). Ces enzymes sont exprimées par certaines cellules d'organismes vivants et peuvent être extraites de ces cellules ou purifiées à partir de cultures recombinantes.

Ces enzymes requièrent l'existence d'un fragment d'acide nucléique initial appelé *amorce.* Ce fragment initial sert de substrat lors du premier cycle d'élongation des acides nucléiques à synthétiser. Il possède un groupe 3'-OH libre de réagir avec le groupe 5'-OH du premier nucléotide à additionner.

Dans un mode de réalisation préféré les amorces possèdent la longueur la plus propice à la réalisation de la présente méthode ainsi que la séquence la plus favorable au bon déroulement des étapes successives. Si besoin, plusieurs amorces différentes peuvent être utilisées simultanément ou successivement sans limitation de nombre. Cette amorce peut comporter des nucléotides naturels ou des nucléotides modifiés. Les nucléotides employés sont généralement constitués d'un sucre cyclique comportant au moins un groupe chimique à l'extrémité 5' et un groupe chimique à l'extrémité 3' et d'une base azotée naturelle ou modifiée.

Les conditions du milieu réactionnel, notamment la température, la pression, le pH, un milieu tamponné éventuel, et autres réactifs, sont choisies pour permettre un fonctionnement optimal des enzymes de polymérisation tout en garantissant l'intégrité des structures moléculaires des différents réactifs également présents.

L'extrémité libre du ou des nucléotides additionnés aux fragments comporte avantageusement un groupement chimique protecteur, pour empêcher une addition multiple des X nucléotides sur un même fragment.

L'addition de nucléotides protégés à leur extrémité 3' constitue l'un des modes de réalisation préféré de la présente méthode le groupement protecteur ayant pour but d'empêcher toute possibilité de polymérisation ultérieure limitant ainsi le risque de polymérisation incontrôlée. Outre la prévention de la création d'une liaison phosphodiester, d'autres fonctions, telles que l'interaction avec le second support solide ou l'interaction avec d'autres réactifs du milieu réactionnel peuvent être attribuées audit groupement protecteur.

Par exemple, le second support peut être recouvert de molécules, telles que des protéines, permettant une liaison non covalente entre ces molécules et ledit groupement chimique protecteur, et ainsi l'immobilisation des fragments additionnés lors de la seconde étape d'accrochage. Le nucléotide protégé, et lui seul, est capable d'interagir avec le second support solide. L'élimination des fragments non-additionnés peut ainsi s'effectuer par défaut d'interaction entre leur extrémité 3' et le second support solide.

La seconde phase du présent procédé ne comporte pas d'étape de détection des fragments ayant une séquence erronée. Ces fragments ne sont pas identifiés comme tels et aucune information manipulable ne permet de les identifier afin de les isoler ou de les corriger. Le présent procédé élimine simplement les fragments erronés par défaut d'interaction entre leur extrémité 3' et le second support solide.

L'étape de décrochage desdits fragments corrects à n + Xi nucléotides dudit second support peut ensuite être effectuée par modification des conditions du milieu réactionnel, telles que des modifications du pH, ou de la température, ou sous l'action d'un rayonnement électromagnétique si ledit second support est un support magnétique.

De préférence, la première étape d'accrochage correspond à l'accrochage des extrémités 5' des fragments d'acides nucléiques de départ, ou en cours d'élongation, à un premier support et la seconde étape d'accrochage correspond à l'accrochage des extrémités 3' desdits fragments à n + Xi nucléotides à un second support.

L'addition enzymatique s'effectue avantageusement dans le sens 5' vers 3'.

Après l'étape de purification, la déprotection est nécessaire au bon déroulement de la synthèse. Elle a pour rôle de re-générer, au niveau de l'extrémité 3', un groupe -OH apte à réagir lors de la prochaine phase de polymérisation, et donc de permettre une élongation ultérieure, lors du cycle i + 1 suivant, des fragments d'acide nucléique de séquence n +Xi à extrémité protégée. L'étape de déprotection de ladite extrémité peut notamment mettre en œuvre une réaction chimique, une interaction électromagnétique, une réaction enzymatique et/ou une interaction chimique ou protéique.

De manière avantageuse, l'étape de décrochage des fragments liés au second support solide est concomitante et indivisible de l'étape de déprotection des nucléotides protégés. Réciproquement, toute action permettant de déprotéger les nucléotides venant d'être incorporés dans les fragments en cours d'élongation, altère irrémédiablement les capacités d'interaction des fragments avec le second support solide et entraîne par conséquence leur décrochage.

La présente invention met avantageusement en œuvre un mode de synthèse orthogonal. Dans ce mode de synthèse certains réactifs possèdent des propriétés qui leur permettent de ne pas subir certaines étapes alors que les autres réactifs les subiront. Plus particulièrement, le procédé de synthèse selon la présente invention peut être qualifié de "double-orthogonal", c'est-à-dire selon deux "dimensions" : la première dimension concerne l'extrémité 5' des fragments en cours d'élongation liée à un premier support solide, et la seconde dimension concerne l'extrémité 3' desdits fragments liés ensuite à un second support solide.

Les amorces fragments d'acides nucléiques de départ possèdent ainsi des fonctionnalités au niveau de leurs extrémités 5' leur permettant d'interagir avec le(s) premier(s) supports solides ou des éléments immobilisés sur ces dits premiers supports. Les fonctionnalités des amorces sont conservées durant le cours de la synthèse. Les interactions sont réversibles notamment par application de conditions physico-chimiques spécifiques. Il est également possible de neutraliser de façon irréversible ces caractéristiques d'interactions par réaction chimique ou enzymatique.

Les amorces constituent le point de départ de la synthèse en recevant le premier nucléotide à ajouter. Elles sont conservées au cours de cette même synthèse jusqu'à l'ajout du dernier nucléotide présent dans la séquence désirée. Les fonctionnalités présentes sur l'extrémité 5' de ces amorces sont avantageusement choisies de façon à rester fonctionnelles tout au long de la synthèse. Par conséquent ces fonctionnalités confèrent aux fragments d'acide nucléique en cours d'élongation les mêmes caractéristiques et propriétés que celles initialement conférées aux amorces, l'aptitude à s'accrocher, lors de chaque cycle d'élongation, au premier support solide, préalablement à l'étape d'addition enzymatique, par l'intermédiaire de brins d'acides nucléiques déjà fixés audit premier support.

Dans le mode de réalisation préféré de la méthode les fonctionnalités présentes à l'extrémité 5' des amorces utilisées pour l'initiation de la synthèse permettent des interactions non-covalentes avec des molécules ou des protéines présentes sur ledit premier support solide. Lors de chaque étape de la synthèse ces fonctionnalités sont préservées car ne présentant pas de réactivité avec les réactifs employés. Les liaisons non-covalentes étant suffisamment fortes pour assurer des interactions stables selon certaines conditions physico-chimiques mais également suffisamment faibles pour être supprimées en cas d'application de conditions physico-chimiques différentes. Enfin la structure moléculaire de ces fonctionnalités peut leur conférer la possibilité d'être détruites ou supprimées par action de réactifs spécifiques, cette propriété pouvant être utile dans les cas où l'on souhaite la destruction ou suppression des nucléotides constituant les amorces initiales.

Les surfaces desdits supports solides possèdent des fonctionnalités leur permettant l'interaction recherchée avec les fonctionnalités présentes sur les amorces. Comme ces dernières, les fonctionnalités de ces supports solides doivent pouvoir subir les différentes étapes du cycle de synthèse sans dégradation de leurs performances d'interactions.

L'application de certaines conditions physico-chimiques telles que, variation de température, de pH et/ou de champ électromagnétique peut permettre de mettre fin aux interactions existantes entre les fragments d'acide nucléique en cours de synthèse et les supports solides. Dans d'autres cas l'application de conditions physico-chimiques distinctes de celles décrites précédemment doit permettre un renforcement de ces interactions. Les différentes conditions physico-chimiques sont appliquées aux moments opportuns des cycles de synthèse des fragments d'acide nucléique.

Plus particulièrement, des conditions physico-chimiques renforçant les interactions entre le(s) premier(s) support(s) solide(s) et les extrémités 5' des fragments sont appliquées durant toutes les étapes de synthèse exceptée lors des étapes de décrochage des fragments d'acide nucléique. Lors de ces étapes de décrochage les conditions physico-chimiques sont modifiées afin de libérer les fragments.

Entre deux phases de chaque cycle d'élongation, les fragments d'acide nucléique détachés des supports sont déplacés par création d'un flux mobile de fluide, au moyen de pompes ou tout autre dispositif permettant de déplacer des fluides. Plus particulièrement, les fragments sont alors détachés desdits supports par un flux de liquide apportant les conditions physico-chimiques favorables à l'annulation de l'interaction entre les fragments d'acide nucléique et lesdits supports. Les fragments sont alors déplacés alors que les supports restent immobilisés.

Les nucléotides additionnés constituent les entités élémentaires de polymérisation de la synthèse. Ils sont ajoutés les uns après les autres aux fragments d'acide nucléique. Ces nucléotides présentent des fonctionnalités d'interaction en plus de leurs fonctionnalités de réactivité de liaison et de leurs fonctionnalités de protection. Les fonctionnalités d'interaction des nucléotides ont un fonctionnement similaire aux fonctionnalités d'interaction de l'extrémité 5' des amorces.

De préférence les fonctionnalités d'interaction de l'extrémité 5' des fragments d'acide nucléique en cours de synthèse et celles des nucléotides récemment ajoutés sont utilisées conjointement au cours du même cycle de synthèse.

L'application de conditions physico-chimiques spécifiques telles que la température, le pH, l'environnement électromagnétique ou la présence de matériel de filtration, peut être mise en œuvre pour réaliser la séparation entre les différents supports mentionnés ci-dessus et les fragments d'acides nucléiques de séquence correcte ou incorrecte.

Les différentes étapes de la synthèse enzymatique de fragments d'acide nucléique selon l'invention contribuent aux performances de cette synthèse en assurant la polymérisation correcte d'un maximum de fragments d'acide nucléiques, et permettent également, par l'intermédiaire des mécanismes d'interaction précédemment évoqués, l'élimination du plus grand nombre possible de fragments d'acide nucléique ne possédant pas la séquence désirée car ayant échoué à l'une des étapes constituant le cycle d'élongation.

Ainsi, la présente invention définie par les revendications ci-jointes met en œuvre le principe « d'élimination large » de fragments d'acide nucléique. Cette « élimination large » englobe majoritairement les fragments possédant une séquence erronée mais également dans une certaine mesure des fragments possédant une séquence correcte. Le but de ce concept d'élimination large est d'éliminer la totalité des fragments possédant une séquence erronée même si cela se fait au détriment de la conservation de certains fragments possédant une séquence correcte.

De manière générale les étapes d'élimination des impuretés, incluant dans certains cas les fragments ayant une séquence erronée, sont réalisées par des lavages, mettant en œuvre des flux mobiles de fluides. Durant les étapes de lavage les éléments qu'il convient de conserver le sont notamment par interaction avec les supports solides, par exemple maintenus immobiles ou retenus par filtration.

De préférence, dans le procédé selon la présente invention, définie par les revendications ci-jointes les interactions sont choisies de façon à d'être sélectives et donc de n'agir que sur les acides nucléiques avec une séquence correcte et non sur les autres composés présents dans le milieu réactionnel comme par exemple les fragments d'acides nucléiques n'ayant pas réagi, les enzymes ou des composants des solutions tampon. A titre d'exemple, les interactions entre les nucléotides ajoutés et les supports solides sont choisies de sorte que seuls les fragments ayant incorporé les nucléotides protégés soient conservés lors des étapes de lavage. Ces mêmes interactions sont choisies de façon à être suffisamment faibles pour que dans tous les cas seuls les fragments ayant incorporé les nucléotides protégés soient conservés lors des étapes de lavage. De telles interactions faibles peuvent avoir pour conséquence de ne pas conserver, dans une certaine proportion, les fragments ayant bien incorporé les nucléotides même s'ils devaient être conservés. Cette caractéristique est recherchée pour l'application du procédé selon l'invention dans la mesure où elle assure l'élimination la plus complète possible des fragments d'acide nucléiques non désirés.

Les interactions non-covalentes précédemment décrites sont choisies de telle sorte qu'elles permettent une interaction efficace avec une proportion des fragments désirés et une interaction nulle avec les fragments non désirés, notamment par optimisation de la structure des entités interagissant entre elles et optimisation des conditions physico-chimique responsable de l'interaction. A titre d'exemple non limitatif, les interactions non covalentes utilisées dans le cadre des différentes techniques de chromatographie ou de purification par affinité permettent de telles performances.

Le procédé de synthèse faisant l'objet de la présente description est également particulièrement adapté à la synthèse simultanée de fragments d'acide nucléique. Un cycle de synthèse permettant l'accroissement d'un nucléotide de la séquence désirée, peut être divisé en plusieurs étapes réalisables en parallèle pour la synthèse simultanée de plusieurs fragments différents.

Ainsi, sous réserve de ressources suffisantes, il n'y a pas de limitation aux nombres de fragments d'acide nucléique différents pouvant être synthétisés simultanément. Avantageusement la synthèse en parallèle de nombreux fragments peut accroître de façon significative les capacités de synthèse d'acide nucléiques d'un expérimentateur mettant en œuvre la présente invention.

Dans un mode de réalisation préféré de la présente description, en parallèle de la synthèse des fragments désirés, il peut effectuer la synthèse simultanée de la totalité ou d'une partie des fragments complémentaires à ceux désirés.

Dans un autre mode de réalisation préféré, des synthèses peuvent être menées simultanément sur des fragments ayant un lien entre eux. A titre d'exemple, les fragments en question peuvent constituer les portions d'une plus grande séquence d'intérêt comme, mais non limité à, un gène, une région chromosomique, un chromosome ou un génome. Toujours à titre d'exemple, ces différents fragments constituant une séquence commune peuvent avoir des portions de séquence identiques ou complémentaire facilitant un éventuel assemblage ultérieur.

Le procédé selon la présente description inclut une ou plusieurs phases d'amplification des fragments d'acide nucléique de séquence correcte, permettant de multiplier le nombre de ces fragments d'acides nucléiques présents dans le milieu réactionnel. Cette amplification en chaîne d'acides nucléiques utilise une méthode de polymérisation enzymatique combinant des brins d'acides nucléiques matrices, des nucléotides, des amorces initiales complémentaires et des enzymes de polymérisation template-dependant. Le résultat de l'amplification est constitué par une copie en très grand nombre des brins matrices initialement présents. L'amplification est réalisée par une succession d'étapes constituant un cycle d'amplification. Le facteur de multiplication *Y_{M}* des fragments amplifiés est dépendant du nombre de cycles m d'amplification réalisés suivant la formule : Y_{M} = 2^{m}

Dans un mode de réalisation préféré les enzymes utilisés pour l'amplification sont capables d'assurer la polymérisation des nucléotides par copie du brin matrice. De telles enzymes sont alors dépendantes d'un brin complémentaire. Ces enzymes ont la capacité de synthétiser des fragments d'acide nucléique double brin.

Des exemples, non limitatifs pour la présente invention, d'enzymes utilisables pour la phase d'amplification sont les ADN-polymérases ADN-dépendante. Ces enzymes sont disponibles commercialement et sont souvent purifiées à partir de cultures recombinantes.

Les nucléotides employés pour l'amplification sont généralement des nucléotides naturels comportant au moins un groupe chimique à l'extrémité 5' de type triphosphate et un groupe chimique à l'extrémité 3' de type hydroxy et d'une base azotée naturelle. Dans un mode de réalisation préférée, les nucléotides employés pour l'amplification sont des nucléotides naturels.

Les conditions réactionnelles telles que température, pression, pH, tampon, cofacteurs et/ou autres réactifs, ainsi que les étapes et leur enchainement sont choisis de façon à assurer une amplification optimale des fragments considérés.

L'amplification nécessite la présence d'amorces complémentaires aux fragments d'acide nucléique à amplifier. Les amorces complémentaires sont des fragments d'acide nucléique de faible longueur possédant une séquence de nucléotide complémentaire de celle des fragments à amplifier. Ils sont de ce fait constitués de nucléotides similaires et pouvant interagir avec ceux constituant les fragments à amplifier. Ces amorces complémentaires peuvent avantageusement être synthétisées en parallèle des fragments d'acides nucléiques synthétisés selon le procédé de la présente invention. Ainsi il est possible selon ce principe d'amplifier n'importe quelle séquence considérée sans faire appel à un quelconque procédé différent de celui objet de la présente invention.

Les amorces complémentaires doivent interagir de façon non covalente avec une des extrémités des fragments à amplifier pour que l'enzyme d'amplification puisse réagir. Ces interactions non covalentes sont dépendantes et spécifiques des séquences complémentaires des fragments à amplifier et des amorces. Ainsi la spécificité de ces interactions limite de façon très importante l'amplification des éventuels fragments ne possédant pas une séquence correcte à leur extrémité 3'. De cette façon l'étape d'amplification contribue également à la pureté du résultat final du procédé de synthèse.

### Acides nucléiques synthétisés

La présente description concerne également les acides nucléiques synthétisés par le procédé décrit ci-dessus. La séquence nucléotidique de ces acides nucléiques peut être prédéterminée, imposant l'ordre de polymérisation desdits nucléotides lors de la réalisation des cycles d'élongation. Il est cependant envisageable d'utiliser le présent procédé pour la synthèse d'acides nucléiques ayant des séquences aléatoires.

Le présent procédé de synthèse permet la synthèse d'acides nucléiques de toutes tailles, la taille minimum des acides nucléiques étant néanmoins déterminée par la taille minimum des amorces initiales utilisées au cours de la synthèse. Sous réserve de ressources disponibles en quantités suffisantes et sous réserve d'espace et de conditions favorables, il n'y a pas de taille maximale d'acides nucléiques synthétisables par le présent procédé. Dans un mode de réalisation préféré de la présente invention, il est possible de synthétiser des fragments d'acide nucléiques ayant une longueur comprise entre 3 et 1.10⁹ bases, de préférence entre 20 et 1.10⁷ bases.

Les acides nucléiques ainsi synthétisés peuvent comporter des séquences nucléotidiques ayant de multiples rôles biologiques ou biotechnologiques. Avantageusement, la présente description remplace un certain nombre de manipulations du domaine de la biologie moléculaire et de l'ingénierie génétique, ces manipulations fastidieuses et répétitives étant réalisées habituellement à la main par des expérimentateurs de l'art.

L'utilisation du procédé de la présente description permet une augmentation significative des performances et de la productivité de tout procédé mettant en jeu des manipulations d'acides nucléiques. A titre d'exemples, non limitatifs, l'utilisation de la présente invention est particulièrement avantageuse dans les domaines suivants: élaboration de constructions génétiques, production de molécules d'ARN interférant, production de puce à ADN ou ARN, construction de souches ou lignées cellulaires, ingénierie enzymatique, développement de modèles protéiques, développement de biothérapies, développement de modèle animaux ou végétaux.

Les acides nucléiques obtenus à l'aide du procédé décrit ci-dessus peuvent être directement utilisés au cours de manipulations de biologie moléculaire connus des hommes de l'art. Les acides nucléiques ainsi obtenus présentent un taux de pureté extrêmement bien adapté à une utilisation directe sans besoin d'étapes de traitement supplémentaires. En variante, les acides nucléiques synthétisés par procédé selon l'invention de synthèse peuvent subir des modifications complémentaires visant par exemple, à circulariser les fragments d'acides nucléiques, insérer les fragments d'acides nucléiques dans des vecteurs d'expression, insérer les fragments d'acides nucléiques dans le génome de cellules vivantes, faire réagir les fragments d'acides nucléiques avec d'autres entités chimiques ou utiliser les fragments d'acides nucléiques pour la catalyse d'une réaction.

### Automatisation

L'automatisation du procédé objet de la présente description peut être réalisée par n'importe quel dispositif préalablement adapté pouvant optimiser celui-ci notamment en minimisant la durée d'un cycle d'élongation, en maximisant la précision des ajouts, lavages et flux de fluides et en optimisant les conditions réactionnelles employées lors des différentes étapes de ces cycles.

Le procédé selon la présente description permet la synthèse d'acides nucléiques directement utilisables sans étapes de purification ou d'assemblage supplémentaires du fait de la grande pureté des acides nucléiques synthétisés et cela indépendamment de leur taille. L'automatisation du procédé objet de la présente description possède de ce fait un grand intérêt industriel et commercial.

### Kits

La présente invention définie par les revendications ci-jointes concerne également tout kit pour la mise en œuvre du procédé décrit ci-dessus, comprenant :
- un milieu réactionnel renfermant des fragments d'acides nucléiques comportant n nucléotides
- un réactif enzymatique d'addition de nucléotides
- des nucléotides ou combinaisons de nucléotides aptes à être ajoutés par ledit réactif enzymatique d'addition
- des solutions de lavage et/ou tampon pour la phase de purification
- un milieu réactionnel d'amplification renfermant : un réactif enzymatique d'amplification d'acides nucléiques, et des nucléotides naturels aptes à être utilisés par le réactif enzymatique d'amplification
- une notice d'utilisation.

Différents types de kits peuvent être proposés suivant les besoins de l'expérimentateur. En particulier différentes amorces d'initiation peuvent être proposés dans différents kits en fonctions des séquences à synthétiser. De façon similaire, différents types de kits peuvent être proposés en fonction d'une utilisation automatisée ou non.

Selon un mode de réalisation préféré le kit pour la mise en œuvre du mode de réalisation préféré du procédé comprend :
- des fragments d'acide nucléiques utilisés comme amorce de synthèse.
- un premier support d'accrochage des fragments d'acides nucléiques comportant n nucléotides
- un second support d'accrochage des fragments d'acides nucléiques additionnés
- un milieu réactionnel d'addition renfermant : un réactif enzymatique d'addition de nucléotides et des nucléotides ou combinaisons de nucléotides aptes à être ajoutés par le réactif enzymatique d'addition
- des solutions *de lavage et*/*ou tampon* pour les étapes d'accrochage, de purification et de décrochage
- un milieu réactionnel d'amplification renfermant : un réactif enzymatique d'amplification d'acides nucléiques, et des nucléotides naturels aptes à être utilisés par le réactif enzymatique d'amplification
- une notice d'utilisation.

Le kit ci-dessus peut en outre comprendre :
- des milieux tampons favorables à l'accrochage des fragments d'acide nucléiques sur le premier et/ou le second support d'accrochage, et/ou
- des milieux tampons favorables au décrochage des fragments d'acides nucléiques du premier et/ou second support d'accrochage.

### Description des figures

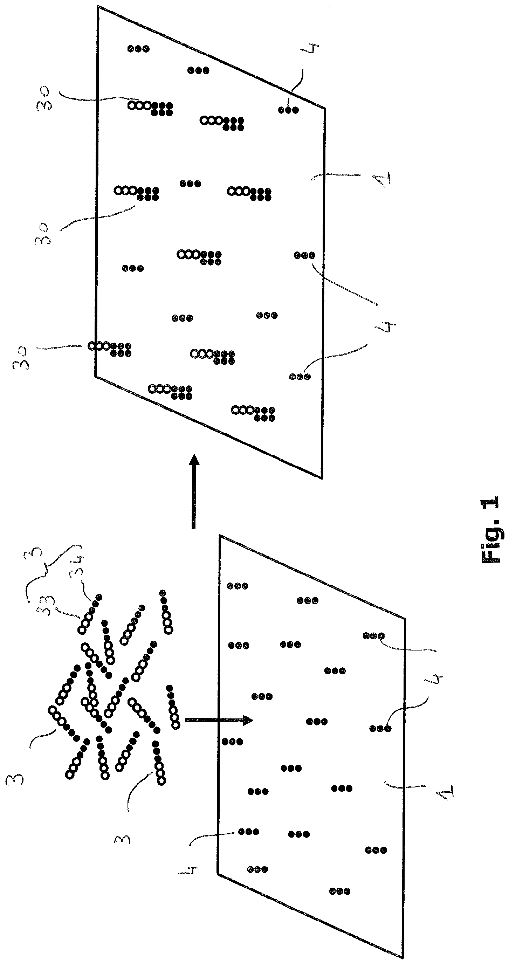

La description d'un exemple de réalisation du mode préféré du présent procédé avec mise en œuvre de supports d'accrochage pour immobiliser les fragments d'acides nucléiques lors de certaines étapes du procédé de synthèse va être décrite ci-après en référence aux figures dans lesquelles les figures 1 à 7 schématisent les différentes étapes du procédé :
la figure 1 l'immobilisation des fragments de départ ;
la figure 2 l'addition d'un nucléotide aux fragments immobilisés ;
la figure 3 une étape de lavage et le décrochage des fragments d'acides nucléiques additionnés ;
la figure 4 la fixation, sur un second support, des fragments d'acides nucléiques additionnés ;
la figure 5 la déprotection des fragments d'acides nucléiques additionnés ;
la figure 6 une étape d'amplification ;
la figure 7 le démarrage d'un nouveau cycle en utilisant les fragments additionnés du cycle précédent ;
et la figure 8 présente un schéma d'agencement possible des "réacteurs" pour la réalisation de la phase d'addition enzymatique et de la phase de purification des fragments de séquence correcte.

### Exemple

En référence aux figures, un cycle d'élongation de fragments d'acides nucléiques dans le procédé de synthèse est décrit ci-après.

Sur un premier support solide 1, tel qu'une plaque de verre, sont fixés des brins 4 comportant au moins trois nucléotides. Des amorces ou des fragments en cours d'élongation 3 d'acides nucléiques renfermant Xi + n nucléotides sont liés aux brins 4 fixés sur le support 1 par l'intermédiaire de liaisons hydrogène entre leurs bases respectives. Le fragment d'acide nucléique en cours d'élongation 3 comporte une partie libre 33 et une partie immobilisable 34 à au moins trois nucléotides complémentaires de ceux des brins fixés 4 et représentant l'amorce. On obtient alors, dans le milieu réactionnel, comme schématisé sur la droite de la figure 1, des fragments d'acides nucléiques 30 immobilisés sur le premier support solide 1.

On réalise ensuite une étape d'addition enzymatique par l'ajout d'un milieu réactionnel renfermant des enzymes d'addition 5 et des réactifs 6 comportant au moins un nucléotide 7 dont une extrémité est bloquée par un groupement protecteur 8.

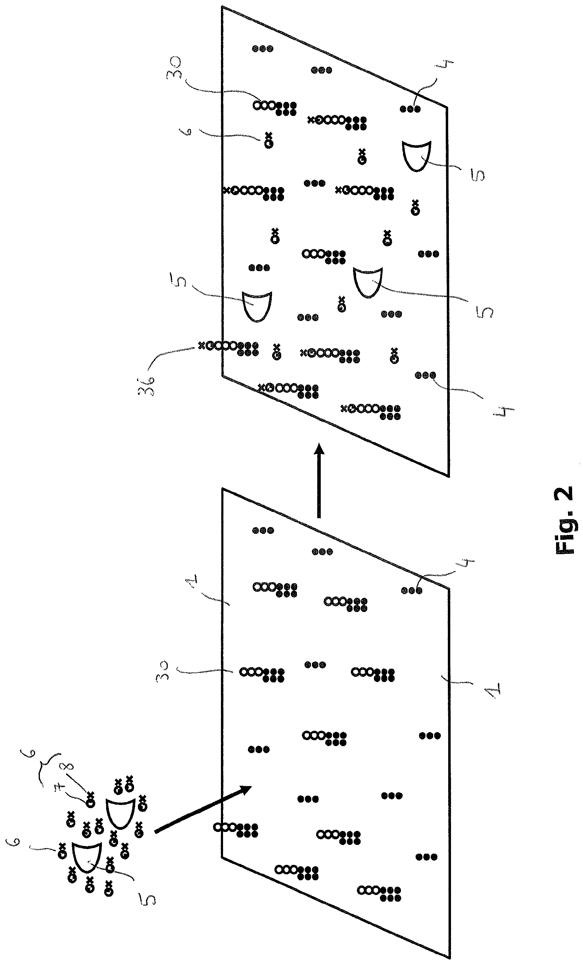

Il en résulte, comme schématisé sur la partie droite de la figure 2, l'addition des réactifs 6 à l'extrémité libre 33 des fragments 30 immobilisés sur le premier support solide pour donner des fragments d'acides nucléiques additionnés (c'est-à-dire ayant reçu au moins un nucléotide) à extrémité protégée, immobilisés sur le support 1. Ces fragments d'acides nucléiques protégés et immobilisés sont référencés 36.

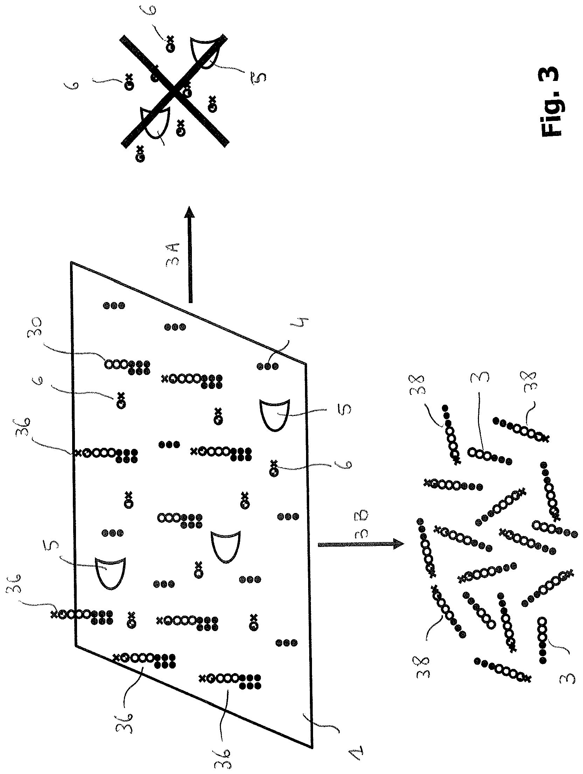

Il subsiste, dans le milieu réactionnel, soit sur le support, soit à proximité dudit support 1, des résidus d'enzymes, de réactifs 6, et d'éventuelles solutions tampons, qui sont alors éliminés par lavage selon la flèche 3A. Après ce premier lavage 3A, sont décrochés, selon la flèche 3B, du support 1 les fragments 36 qui vont donner des fragments additionnés, protégés, libres référencés 38 sur la figure 3.

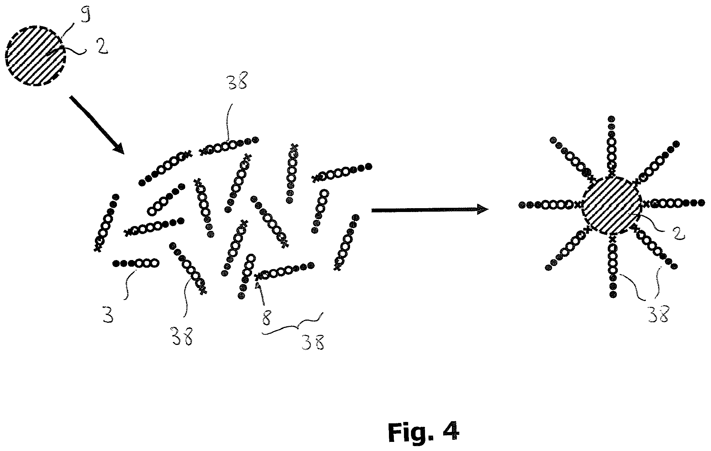

Cependant, cette étape de décrochage détache du support 1 à la fois les fragments additionnés 38 et les fragments initiaux 3 non additionnés. On fait alors intervenir, comme schématisé sur la figure 4, un second support 2, ici des billes magnétiques, recouvertes d'un revêtement 9 formé de protéines telles que des anticorps, la dihydrofolate réductase (DHFR), l'avidine, la streptavidine, la glutathionne-S-transférase (GST), des phosphopeptides (oligomères de sérine, tyrosine ou thréonine) ou des oligomères d'histidine dans le milieu réactionnel renfermant les fragments d'acides nucléiques de séquence correcte et les fragments de séquence incorrecte.

Il en résulte une fixation par le groupement protecteur terminal 8 un accrochage, à la surface des billes 2, des fragments de séquence correcte protégés 38. Des fragments 3 initiaux n'ayant pas subi l'addition enzymatique, et donc ne comportant pas d'extrémité avec le groupement protecteur pouvant se lier aux protéines du revêtement 9 des billes 2, sont alors aisément éliminés par lavage.

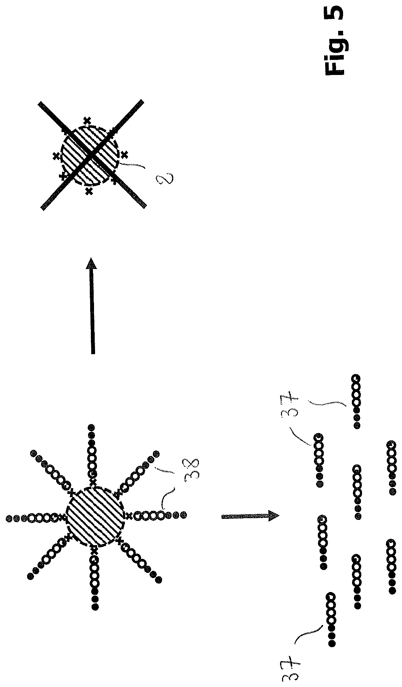

Après cette étape de sélection efficace des fragments additionnés de séquence correcte, les billes magnétiques 2 sont séparées par modification des conditions du mélange réactionnel. Par exemple une modification du pH, une élévation de la température, ou l'action d'un réactif ou d'un champ électromagnétique permet de décrocher des billes 2 les fragments additionnés pour obtenir des fragments additionnés non protégés 37 libres dans le milieu réactionnel (voir figure 5).

Les billes 2 recouvertes des groupements protecteurs restés liés au revêtement 9 sont alors éliminées du milieu réactionnel, par exemple sous l'action d'un champ magnétique.

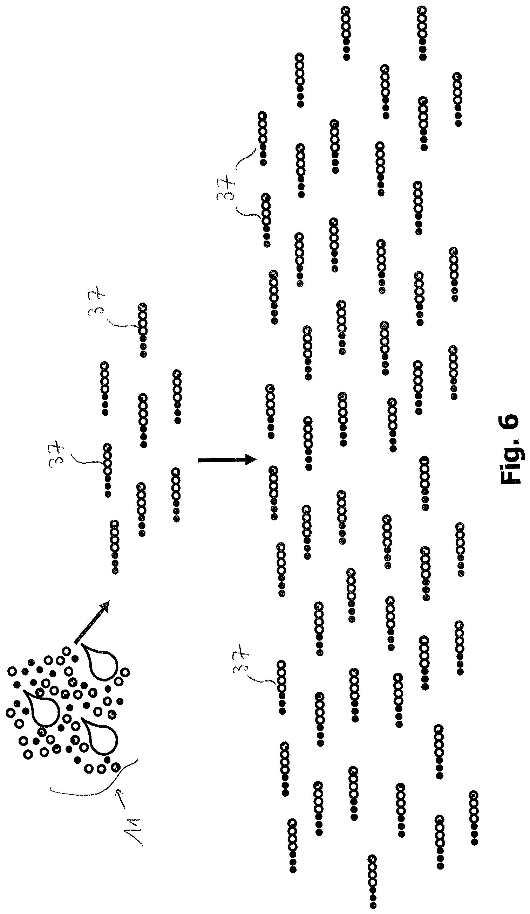

La figure 6 schématise ensuite une étape d'amplification des fragments additionnés non protégés 37 ayant subi le cycle d'élongation décrit jusqu'à présent, sous l'action d'un milieu réactionnel d'amplification 11 comportant les enzymes d'amplification ainsi que les nucléotides, par exemple naturels. Le nombre des fragments d'acides nucléiques 37 est alors amplifié considérablement.

Cette étape d'amplification est tout à fait efficace car elle n'amplifie que les fragments 37 ayant subi le cycle d'élongation. Ceci minimise en outre l'emploi des réactifs d'amplification.
Aucune étape ultérieure de purification n'est alors nécessaire, avant de terminer la synthèse ou de procéder à un nouveau cycle i+1 d'élongation.

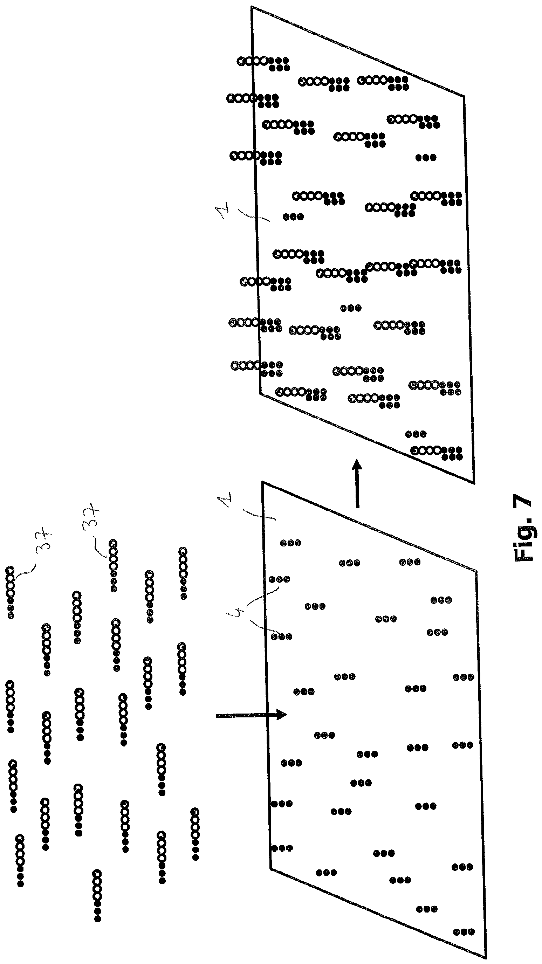

Un nouveau cycle d'élongation peut alors intervenir par le biais du même premier support solide 1 sur lequel sont fixés les brins 4 servant à immobiliser les fragments 37, comme schématisé sur la figure 7.

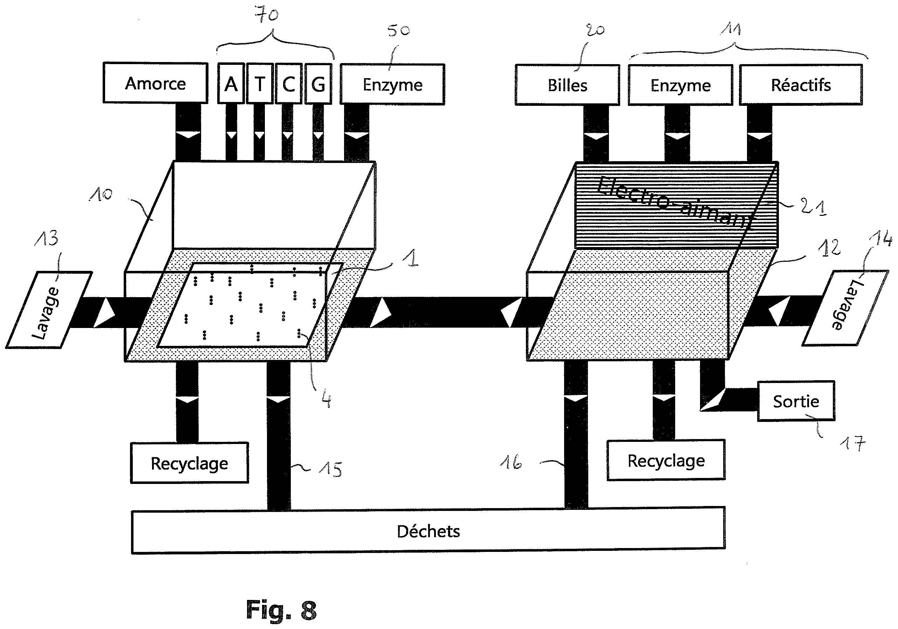

La figure 8 présente le schéma d'un exemple de chambre d'élongation, de préférence à deux compartiments distincts, dans lesquels est réalisé chaque cycle d'élongation

Le premier réacteur 10, compartiment dans lequel est réalisée la phase d'addition enzymatique, renferme le premier support 1 solide sur lequel sont fixés les brins 4, et est connecté à un dispositif d'alimentation 70 en nucléotides 7 d'addition et à un dispositif d'alimentation 50 en enzyme d'addition 5.

Le second réacteur 12, compartiment dans lequel est réalisée la phase de purification des fragments de synthèse correcte, est équipé ici d'un dispositif électromagnétique (électroaimant 21) et est connecté à une alimentation 20 en supports de type billes.

Les deux réacteurs 10 et 12, séparés pour permettre une meilleure purification des fragments sont néanmoins connectés entre eux et connectés à des entrées 13, 14 de solution(s) de lavage ainsi qu'à des sorties 15, 16 de déchets à évacuer.

La ou les étape(s) d'amplification est(sont) également effectuée(s) à l'intérieur du réacteur 12.

Les acides nucléiques ainsi synthétisés sont récupérés à la sortie 17 du second réacteur après l'étape finale d'amplification.

Un exemple illustratif de synthèse d'un fragment d'ADN est décrit ci-après.

L'enzyme choisie pour la réalisation des étapes de synthèse enzymatique est la terminale déoxynucléotidyl transférase ou TdT disponible commercialement (Thermo Scientific). Il est également possible de produire de manière recombinante des quantités importantes de TdT.

L'amorce utilisée pour initier la synthèse est donnée ci-dessous :

| | |
|---|---|
| 5'-GGGGGGGGGGGGGGCTGCA^{↓}G-3' | Seq N°1 |

Cette amorce possède un site de restriction impliquant une coupure de la séquence par l'enzyme de restriction Pst1 au niveau de la flèche présente dans la séquence ci-dessus. Dans le cas où la séquence à synthétiser comporterait également un site Pst1 l'amorce initiale serait modifiée à façon.

Les nucléotides utilisés possèdent à leur extrémité 5' un groupe triphosphate favorisant leur réactivité. Ils possèdent une des quatre bases azotées naturellement présentes dans la molécule d'ADN à savoir A Adénine, T Thymine, C Cytosine ou G Guanine, et possèdent à leur extrémité 3' un groupe différent du groupe hydroxy naturellement présent et qui possède la capacité à bloquer toute addition ultérieure de nucléotide par la TdT et la capacité à interagir avec d'autres molécules ou protéines.

Les conditions de synthèse utilisées proviennent de la description du protocole associé à l'enzyme TdT : 50 U de TdT, 200 mM sodium cacodylate, 25 mM Tris-HCl pH 7,2, 8 mM MgCl2, 0,33 mM ZnSO4, 0,2 mM dATP, 2 pmol de amorce (Seq N°1) et 100µM de nucléotide protégés sont mélangés pour un total de 50 µl de volume réactionnel. Le mélange est incubé pendant 5 min à 37°C.

Suivant la nature du groupe protecteur choisi, les nucléotides ajoutés sont déprotégés par action d'acide doux tel que l'acétate de sodium 50 mM pH 5,5 en présence de 10 mM MgCl₂ pendant 15 min à 37°C. La réaction de déprotection a pour résultat de détruire la liaison existant entre le nucléotide et le groupe protecteur ainsi que tout autre groupe éventuellement associé au groupe protecteur. Selon certains modes de réalisation cette étape de déprotection peut simultanément libérer le fragment d'une interaction au niveau de son extrémité 3'.

Les fragments d'ADN en cours de synthèse sont incubés pendant 10min à 20°C dans une chambre réactionnelle comportant une plaque de verre sur laquelle ont été fixé au préalable des fragments d'ADN ayant la séquence suivante :

| | |
|---|---|
| 3'-CCCCCCCCCCCCCCGACGTC-5' | Seq N°2 |

Les techniques de génération de puce à ADN ont été mises en oeuvre pour la fixation par l'extrémité 3' de fragment d'ADN possédant la séquence (Seq N°2). Les fragments d'ADN sont donc immobilisés de cette façon.

Les étapes de lavage sont réalisées avec une solution de Tris-HCl 25 mM à pH 7,2 selon un débit de 5 µL par seconde pendant 30 secondes.

Les étapes de libération des fragments d'ADN sont réalisées par passage d'une solution de Tris-HCl 25 mM, pH 7,2 à 95°C au débit de 5 µL par seconde pendant 60 secondes.

Les transferts des fragments d'ADN libres sont réalisés au moyen d'un système de vannes permettant l'acheminement du flux de Tris-HCl 25 mM, pH 7,2 dans lequel sont dissous les fragments, dans le second réacteur 12.

Les fragments d'ADN ayant subi l'étape d'élongation enzymatique utilisant les nucléotides protégés sont alors incubés pendant 15 min à température ambiante en présence de billes magnétiques 2 présentant à leur surface une molécule ou protéine permettant l'interaction avec le dernier nucléotide ajouté. Des billes magnétiques revêtues de protéine GST sont un exemple non limitatif du type de billes qui peuvent être utilisées.

A l'aide d'un aimant permanent, les billes magnétiques 2 portant les fragments d'ADN sont rendues statiques pendant qu'un flux de Tris-HCl 25 mM, pH 7,2 à un débit de 10 µL par seconde est appliqué à des fin de lavage pendant 30 secondes.

L'étape d'amplification des fragments d'ADN est réalisée par l'enzyme Platinum® Taq DNA Polymerase (Invitrogen) selon le protocole suivant : 10X tampon fourni 1x, dNTP 0,2 mM chaque nucléotide, MgCl2 1,5 mM, amorce complémentaire 0,2 µM, Polymerase 1,0 U pour un volume total de 50 µL.

Les cycles d'amplification utilisés sont donnés par le tableau suivant : incubation à 94°C pendant 60 sec, application de 30 cycles d'amplification avec les étapes suivantes: dénaturation à 94°C pendant 30 s puis appariement à 55°C pendant 30 s puis extension à 72°C pendant 60 s par millier de nucléotides à amplifier, une étape d'extension finale à 72°C pendant 5 min est ajoutée.

L'amorce est choisie en fonction de l'état d'avancement de la synthèse des fragments d'ADN. Elle possède une séquence d'environ 20 nucléotides. Les conditions d'amplification décrites ci-dessus sont modulées de façon à favoriser une amplification très spécifique des fragments capables de s'apparier exactement avec l'amorce complémentaire choisie. La taille de cette amorce complémentaire est d'ailleurs également choisie en fonction de la séquence de façon à favoriser l'amplification la plus spécifique possible.

Selon les principes exposés par la présente invention les performances des différentes étapes ont été mesurées et sont présentées dans la tableau 1 ci-après :

**Tableau 1**

| | Taux de bons fragments | Taux de mauvais fragments |
|---|---|---|
| Addition enzymatique | 80 % | 20 % |
| Purification (Etapes d'élimination large) | 90 % | 2 % |
| Amplification | 90 % | 1 % |

Les étapes d'élimination large par exemple permettent de conserver 90% des fragments ayant une séquence correcte (considérés comme "bons") et seulement 2% des fragments ayant une séquence incorrecte (considérés comme "mauvais").

La répétition des cycles mettant en œuvre ces différentes étapes associées de manières judicieuse permettent la synthèse de fragments de grande longueur dont la pureté finale est donnée par le tableau 2 ci-après (quantité d'amorce initiale de 2 pmol):

**Tableau 2**

| | Elongation | | Purification | | Amplification | | Pureté | |
|---|---|---|---|---|---|---|---|---|
| Cycle | Bons | Mauvais | Bons | Mauvais | Bons | Mauvais | Bons | Mauvais |
| 0 | 9,63E+11 | 2.40E+11 | 8,67E+11 | 4,82E+09 | 8,67E+11 | 4,82E+09 | 99,44% | 0,552% |
| 1 | 6,93E+11 | 1,78E+11 | 6,24E+11 | 3,57E+09 | 6,24E+11 | 3,57E+09 | 99,43% | 0,568% |
| 10 | 3,60E+10 | 9,27E+9 | 3,24E+10 | 1,86E+08 | 3,24E+10 | 1,86E+08 | 99,43% | 0,568% |
| 50 | 70870 | 18224 | 63783 | 365 | 63783 | 365 | 99,43% | 0,569% |
| 100 | 4609640 | 1185337 | 4148676 | 23707 | 4148676 | 23707 | 99,43% | 0,568% |

| | Elongation | | Purification | | Amplification | | Pureté | |
|---|---|---|---|---|---|---|---|---|
| Cycle | Bons | Mauvais | Bons | Mauvais | Bons | Mauvais | Bons | Mauvais |
| 500 | 1816 | 467 | 1634 | 10 | 1634 | 10 | 99,392% | 0,608% |
| 1000 | 2852 | 734 | 2566 | 15 | 2566 | 15 | 99,419% | 0,581 % |
| 5000 | 94719 | 24356 | 85247 | 488 | 7,67E+13 | 4,88E+09 | 99,994% | 0,006% |

Ainsi malgré l'imperfection inhérente à chaque étape prise séparément et malgré la présence de fragments échouant à diverses étapes de la synthèse, le résultat final présente une pureté de plus de 99% et est donc utilisable directement par l'expérimentateur et cela de façon indépendante de la longueur du fragment considéré. Les résultats donnés par ce tableau sont présentés à titre d'exemples et peuvent être sensiblement différents si les paramètres (présentés dans le tableau 1) de taux de bons et de mauvais fragments générés à chaque étape, pris comme paramètres, sont différents.

### Application industrielle

Le procédé objet de la présente invention permet la synthèse d'acides nucléiques sans perte de rendement, indépendamment de leur longueur. Il permet une amélioration substantielle des performances de synthèse d'acides nucléiques par rapport aux techniques existantes notamment en termes de simplicité de mise en oeuvre, de coûts de synthèse, de temps de synthèse, de pureté des produits obtenus et de capacité de synthèse. Ce procédé peut être utilisé pour la production de gènes ou de séquences d'acides nucléiques synthétiques. Il est particulièrement destiné à la synthèse d'acides nucléiques tels que l'ADN ou l'ARN à des fins de recherche, de développement ou d'industrialisation dans le domaine de la biotechnologie ou plus généralement dans le domaine large de la biologie.

## Revendications

1. Procédé de synthèse d'acides nucléiques comprenant au moins un cycle d'élongation de fragments d'acides nucléiques, dénomés fragments initiaux comprenant n nucléotides, n allant de 3 à 10¹², chaque cycle se subdivisant de la manière suivante :
a) une phase d'addition enzymatique de Xi nucléotides à une extrémité desdits fragments, X pouvant être compris entre 1 et 5, de préférence entre 1 et 3, i étant le numéro du cycle, permettant d'obtenir des fragments comprenant n + Xi nucléotides, et comprenant les étapes suivantes :
- une première étape d'accrochage, sur un premier support, de l' extrémité 5' de fragments d'acides nucléiques initiaux ou en cours d'élongation, renfermant n nucléotides, ledit premier support présentant une surface d'accrochage permettant une liaison réversible avec l'extrémité 5' desdits fragments,
- une étape d'ajout des réactifs nécessaires à l'addition enzymatique, une étape d'addition enzymatique de Xi nucléotides à la seconde extrémité desdits fragments d'acides nucléiques, l'extrémité libre du ou des nucléotides additionnés aux fragments comporte un groupement chimique protecteur, pour empêcher une addition multiple des Xi nucléotides sur un même fragment,
- une étape de décrochage dudit premier support desdits fragments à n + Xi nucléotides,
une première étape de transfert des dits fragments à n + Xi nucléotides, par création d'un flux mobile de fluide, vers un second support apte à interagir avec le groupement protecteur de l'extrémité 3' des fragments à n + Xi nucléotides au moyen d'une liaison non covalente,
b) une phase de purification des fragments ayant une séquence correcte comprenant n + Xi nucléotides, comprenant les étapes successives suivantes :
- une seconde étape d'accrochage, sur ledit second support, desdits fragments à n + Xi nucléotides par leur extrémité 3',
- une étape d'élimination des fragments non-additionnés et des fragments non-accrochés sur le second support,
- une étape de décrochage desdits fragments à n + Xi nucléotides dudit second support
chaque cycle étant réalisé dans un milieu réactionnel compatible avec une addition et une élongation enzymatiques, le procédé de synthèse comportant également à la fin de l'ensemble des i cycles d'élongation, une étape d'amplification finale d'un facteur multiplicateur Yf.

2. Procédé selon la revendication 1, **caractérisé en ce que** la phase de purification b) comprend l'élimination des réactifs résiduels indésirables.

3. Procédé selon la revendication 1 ou 2, **caractérisé en ce que** la phase d'amplification est une amplification sélective des fragments de séquence correcte à n + xi nucléotides, multipliant dans le milieu réactionnel les fragments ayant une séquence correcte par un facteur multiplicatif plus de 10 fois supérieur, de préférence plus de 100 fois supérieur, au facteur multiplicatif des autres fragments de séquence incorrecte.

4. Procédé selon l'une des revendications 1 à 3, **caractérisé en ce que** le premier support et le second support présentent des surfaces d'accrochage de natures différentes,

5. Procédé selon l'une quelconque des revendications précédentes, **caractérisé en ce que** le premier support présente une surface sur laquelle sont fixés, de manière non covalente, des brins d'acides nucléiques à au moins trois nucléotides, lesdits nucléotides de ces brins étant complémentaires des nucléotides présents à la première extrémité des fragments initiaux, de manière à immobiliser par des liaisons hydrogène entre leurs bases respectives, les dites premières extrémités des fragments.

6. Procédé selon l'une quelconque des revendications précédentes, **caractérisé en ce que** lesdits premier et second supports sont sous la forme de plaque de verre, de plaque en matériau polymère, ou de billes, de préférence l'un ou l'autre de ces supports, avantageusement le second support, présentant des propriétés magnétiques.

7. Procédé selon l'une quelconque des revendications précédentes, **caractérisé en ce que** l'addition de X nucléotides est effectuée au moyen d'enzymes aptes à polymériser des nucléotides modifiés sans présence de brin matrice.

8. Procédé selon la revendication 7, **caractérisé en ce que** l'enzyme est choisie parmi des enzymes de la famille des télomèrases, des enzymes de translésion, des ARN polymérase matrice indépendante ou des enzymes de la famille des terminal déoxynucléotidyl transférases.

9. Procédé selon l'une des revendications 1 à 8, **caractérisé en ce que** le second support est recouvert de molécules, telles que des protéines, permettant une liaison non covalente entre ces molécules et ledit groupement chimique protecteur, et ainsi l'immobilisation des fragments additionnés lors de la seconde étape d'accrochage.

10. Procédé selon l'une quelconque des revendications précédentes, **caractérisé en ce que** l'étape de décrochage desdits fragments à n + Xi nucléotides dudit second support est effectuée par modification des conditions du milieu réactionnel, telles que des modifications du pH, ou de la température, ou sous l'action d'un rayonnement électromagnétique si ledit second support est un support magnétique.

11. Procédé selon l'une quelconque des revendications précédentes, **caractérisé en ce que** l'addition enzymatique s'effectue dans le sens 5' vers 3'.

12. Procédé selon l'une quelconque des revendications précédentes, **caractérisé en ce qu'**il comprend c) une phase d'amplification, de préférence enzymatique, telle que par PCR, des fragments ayant une séquence correcte à n+Xi nucléotides phase, comprenant les étapes successives suivantes :
- une étape d'ajout des réactifs nécessaires à l'amplification,
- une étape de multiplication d'un facteur multiplicateur Yi des fragments à n + Xi nucléotides, i étant le numéro de cycle, Y pouvant être compris entre 1 et 4.10¹⁰, de préférence entre 1 et 1.10⁹.

13. Utilisation du procédé selon l'une quelconque des revendications précédentes pour la production de gènes, ou de séquences d'acides nucléiques synthétiques, d'ADN ou d'ARN.

14. Kit pour la mise en oeuvre du procédé selon l'une quelconque des revendications 1 à 12 comprenant :
- un milieu réactionnel renfermant des fragments d'acides nucléiques comportant n nucléotides
- un réactif enzymatique d'addition de nucléotides
- des nucléotides ou combinaisons de nucléotides aptes à être ajoutés par ledit réactif enzymatique d'addition, lesdits nucléotides comprenant à l'extrémité 3' un groupement chimique protecteur,
- des solutions de lavage (13,14) et/ou tampon pour la phase de purification
- un milieu réactionnel d'amplification renfermant : un réactif enzymatique d'amplification d'acides nucléiques, et des nucléotides naturels aptes à être utilisés par le réactif enzymatique d'amplification
- un premier support présentant une surface d'accrochage permettant une liaison avec l'extrémité 5' de fragments d'acides nucléiques
- un second support présentant une surface d'accrochage permettant une liaison non covalente avec un groupement protecteur de l'extrémité 3' de fragments d'acides nucléiques
- une notice d'utilisation.

15. Kit selon la revendication 14, comprenant en outre :
- des milieux tampons favorables à l'accrochage des fragments d'acide nucléiques sur le premier et/ou le second support d'accrochage, et/ou
- des milieux tampons favorables au décrochage des fragments d'acides nucléiques du premier et/ou second support d'accrochage.

## Patentansprüche

1. Verfahren zur Synthese von Nukleinsäuren, umfassend wenigstens einen Elongationszyklus von Nukleinsäurefragmenten, bezeichnet als Ausgangsfragmente, mit n Nukleotiden, wobei n von 3 bis 10¹² reicht und jeder Zyklus folgendermaßen unterteilt ist in:
a) eine Phase einer enzymatischen Addition von Xi Nukleotiden an einem Ende besagter Fragmente, wobei X zwischen 1 und 5 sein kann, vorzugsweise zwischen 1 und 3 ist, i die Nummer des Zyklus ist, wobei besagte Phase den Erhalt von Fragmenten mit n + Xi Nukleotiden erlaubt und die folgenden Schritte umfasst:
- einen ersten Schritt der Bindung, an einen ersten Träger, eines 5'-Endes von Ausgangsnukleinsäurefragmenten oder von Nukleinsäurefragmenten im Verlauf der Elongation, umfassend n Nukleotide, wobei besagter erster Träger eine Bindungsfläche aufweist, die eine reversible Bindung mit dem 5'-Ende besagter Fragmente erlaubt,
- einen Schritt der Zugabe von Reagenzien, die für eine enzymatische Addition erforderlich sind, einen Schritt der enzymatischen Addition von Xi Nukleotiden an das zweite Ende besagter Nukleinsäurefragmente, wobei das freie Ende des oder der an die Fragmente addierten Nukleotide eine chemische Schutzgruppe umfasst, um eine mehrfache Addition von Xi Nukleotiden am selben Fragment zu verhindern,
- einen Schritt des Ablösens besagter Fragmente mit n + Xi Nukleotiden von besagtem ersten Träger,
einen ersten Schritt des Transfers der besagten Fragmente mit n + Xi Nukleotiden mittels Erzeugung eines mobilen Flüssigkeitsstroms zu einem zweiten Träger, der für eine Wechselwirkung mit der Schutzgruppe des 3'-Endes der Fragmente mit n + Xi Nukleotiden mithilfe einer nichtkovalenten Bindung geeignet ist,
b) eine Phase der Reinigung der Fragmente mit einer korrekten Sequenz mit n + Xi Nukleotiden, umfassend die folgenden aufeinanderfolgenden Schritte:
- einen zweiten Schritt der Bindung, an besagten zweiten Träger, besagter Fragmente mit n + Xi Nukleotiden mit ihrem 3'-Ende,
- einen Schritt des Entfernens der nichtaddierten Fragmente und der an den zweiten Träger nichtgebundenen Fragmente,
- einen Schritt des Ablösens besagter Fragmente mit n + Xi Nukleotiden von besagtem zweiten Träger,
wobei jeder Zyklus in einem Reaktionsmedium, das mit einer enzymatischen Addition und Elongation kompatibel ist, durchgeführt wird,
wobei das Verfahren am Ende der gesamten i Elongationszyklen auch einen letzten Amplifizierungsschritt eines Multiplikationsfaktors Yf umfasst.

2. Verfahren gemäß Anspruch 1, **dadurch gekennzeichnet, dass** die Phase der Reinigung b) das Entfernen unerwünschter restlicher Reagenzien umfasst.

3. Verfahren gemäß Anspruch 1 oder 2, **dadurch gekennzeichnet, dass** die Phase der Amplifikation eine selektive Amplifikation der Fragmente mit korrekter Sequenz mit n + Xi Nukleotiden ist, die im Reaktionsmedium die Fragmente mit einer korrekten Sequenz mit einem Multiplikationsfaktor vervielfacht, der mehr als 10-mal, vorzugsweise mehr als 100-mal höher ist als der Multiplikationsfaktor der anderen Fragmente mit inkorrekter Sequenz.

4. Verfahren gemäß einem der Ansprüche 1 bis 3, **dadurch gekennzeichnet, dass** der erste Träger und der zweite Träger verschiedenartige Bindungsflächen aufweisen.

5. Verfahren gemäß einem der vorhergehenden Ansprüche, **dadurch gekennzeichnet, dass** der erste Träger eine Oberfläche aufweist, an die Nukleinsäurestränge mit wenigstens drei Nukelotiden nichtkovalent gebunden werden, wobei besagte Nukleotide dieser Stränge zu Nukleotiden komplementär sind, die am ersten Ende der Ausgangsfragmente vorhanden sind, um mithilfe von Wasserstoffbindungen zwischen ihren jeweiligen Basen die besagten ersten Enden der Fragmente zu immobilisieren.

6. Verfahren gemäß einem der vorhergehenden Ansprüche, **dadurch gekennzeichnet, dass** besagter erster und zweiter Träger in Form einer Glas- oder Polymermaterialschicht oder von Beads vorliegen, wobei vorzugsweise einer der beiden Träger, vorteilhaft der zweite Träger, magnetische Eigenschaften aufweist.

7. Verfahren gemäß einem der vorhergehenden Ansprüche, **dadurch gekennzeichnet, dass** die Addition von X Nukleotiden mithilfe von Enzymen erfolgt, die für eine Polymerisierung modifizierter Nukleotide in Abwesenheit eines Matrizenstrangs geeignet sind.

8. Verfahren gemäß Anspruch 7, **dadurch gekennzeichnet, dass** das Enzym aus Enzymen der Familie der Telomerasen, Transläsionsenzymen, matrizenunabhängigen RNA-Polymerasen oder Enzymen der Familie der terminalen Desoxynukleotidyltransferasen ausgewählt ist.

9. Verfahren gemäß einem der Ansprüche 1 bis 8, **dadurch gekennzeichnet, dass** der zweite Träger mit Molekülen beschichtet ist, wie beispielsweise mit Proteinen, die eine nichtkovalente Bindung dieser Moleküle mit besagter chemischer Schutzgruppe erlauben und folglich die Immobilisierung der zugefügten Fragmente während des zweiten Bindungsschritts erlauben.

10. Verfahren gemäß einem der vorhergehenden Ansprüche, **dadurch gekennzeichnet, dass** der Schritt des Ablösens besagter Fragmente mit n + Xi Nukleotiden von besagtem zweiten Träger durch Veränderung des Zustands des Reaktionsmediums wie beispielsweise Änderung des pH oder der Temperatur, oder unter Wirkung elektromagnetischer Strahlung erfolgt, falls besagter zweiter Träger ein magnetischer Träger ist.

11. Verfahren gemäß einem der vorhergehenden Ansprüche, **dadurch gekennzeichnet, dass** die enzymatische Addition von 5' nach 3' erfolgt.

12. Verfahren gemäß einem der vorhergehenden Ansprüche, **dadurch gekennzeichnet, dass** es c) eine Phase der Amplifikation, vorzugsweise enzymatische Amplifikation, wie mittels PCR, der Fragmente mit einer korrekten Sequenz mit n + Xi Nukleotiden umfasst, wobei die Phase die folgenden aufeinanderfolgenden Schritte umfasst:
- einen Schritt der Zugabe von Reagenzien, die für die Amplifikation erforderlich sind,
- einen Schritt der Vervielfachung eines Multiplikationsfaktors Yi der Fragmente mit n + Xi Nukleotiden, wobei i die Nummer des Zyklus ist, Y zwischen 1 und 4 x 10¹⁰, vorzugsweise zwischen 1 und 1 x 10⁹ sein kann.

13. Anwendung des Verfahrens gemäß einem der vorhergehenden Ansprüche zur Herstellung von Genen oder von synthetischen Nukleinsäuresequenzen, von DNA oder von RNA.

14. Kit zur Durchführung des Verfahrens gemäß einem der Ansprüche 1 bis 12, umfassend:
- ein Reaktionsmedium, umfassend Nukleinsäurefragmente mit n Nukleotiden,
- ein enzymatisches Reagens zur Addition von Nukleotiden,
- Nukleotide oder Kombinationen von Nukleotiden, die von besagtem enzymatischen Additionsreagens hinzugefügt werden können, wobei besagte Nukleotide am 3'-Ende eine chemische Schutzgruppe aufweisen,
- Waschlösungen (13, 14) und/oder Pufferlösungen für die Phase der Reinigung,
- ein Reaktionsmedium für die Amplifikation umfassend: ein enzymatisches Reagens zur Amplifikation von Nukleinsäuren und natürliche Nukleotide, die sich für eine Verwendung von dem enzymatischen Amplifikationsreagens eignen,
- einen ersten Träger, der eine Bindungsfläche aufweist, die eine Bindung von Nukleinsäurefragmenten mit dem 5'-Ende erlaubt,
- einen zweiten Träger, der eine Bindungsfläche aufweist, die eine nichtkovalente Bindung von Nukleinsäurefragmenten mit einer Schutzgruppe des 3'-Endes erlaubt,
- eine Gebrauchsanleitung.

15. Kit gemäß Anspruch 14, außerdem umfassend:
- Puffermedien, die eine Bindung der Nukleinsäurefragmente an den ersten und/oder zweiten Träger fördern, und/oder
- Puffermedien, die ein Ablösen der Nukleinsäurefragmente von dem ersten und/oder zweiten bindenden Träger fördern.

## Claims

1. A process for the synthesis of nucleic acids comprising at least one cycle of elongation of nucleic acid fragments, known as initial fragments comprising n nucleotides, n ranging from 3 to 10¹², each cycle being subdivided in the following way:
a) A phase of enzymatic addition of Xi nucleotides to one end of said fragments, it being possible for X to be between 1 and 5, preferably between 1 and 3, i being the cycle number, making it possible to obtain fragments comprising n + Xi nucleotides, and comprising the following stages:
- a first stage of attaching, to a first support, the 5' end of initial nucleic acid fragments or nucleic acid fragments in the course of elongation, including n nucleotides, said first support having an attaching surface allowing a reversible connection with the 5' end of said fragments,
- a stage of addition of the reagents necessary for the enzymatic addition, a stage of enzymatic addition of Xi nucleotides to the second end of said nucleic acid fragments, the free end of the nucleotide(s) added to the fragments has a protective chemical group, to prevent multiple addition of Xi nucleotides to the same fragment,
- a stage of detaching from said first support said fragments comprising n + Xi nucleotides,
a first stage of transfer of said fragments comprising n + Xi nucleotides, by creating a mobile fluid flow, to a second support capable of interacting with the protective group of the 3' end of the fragments comprising n + Xi nucleotides by means of a non-covalent bond,
b) a phase of purification of the fragments having a correct sequence comprising n + Xi nucleotides, comprising the following successive stages:
- a second stage of attaching, to said second support, said fragments comprising n + Xi nucleotides by their 3' end,
- a stage of removal of the fragments which have not been added to and of the fragments which have not been attached to the said second support,
- a stage of detaching said fragments comprising n + Xi nucleotides from said second support
each cycle being carried out in a reaction medium compatible with an enzymatic addition and an enzymatic amplification,
the synthesis process also comprising, at the end of all of the i elongation cycles, a stage of final amplification by a multiplication factor Yf.

2. The process according to claim 1, **characterized in that** the phase of purification b) comprises the removal of undesirable residual reagents.

3. The process according to claim 1 or 2, **characterized in that** the amplification phase is a selective amplification of the correct sequence fragments comprising n + Xi nucleotides, multiplying in the reaction medium the fragments having a correct sequence by a multiplying factor more than 10 times greater, preferably more than 100 times greater, than the multiplying factor of the other incorrect sequence fragments.

4. The process according to any one of claims 1 to 3, **characterized in that** the first support and the second support exhibit attaching surfaces of different natures.

5. The process according to any one of the preceding claims, **characterized in that** the first support exhibits a surface to which are fixed, noncovalently, nucleic acid strands comprising at least three nucleotides, said nucleotides of these strands being complementary to the nucleotides present at the first end of the initial fragments, so as to immobilize, by hydrogen bonds between their respective bases, said first ends of the fragments.

6. The process according to any one of the preceding claims, **characterized in that** said first and second supports are in the form of a glass sheet, of a sheet of polymer material, or of beads, preferably one or other of these supports, advantageously the second support, exhibiting magnetic properties.

7. The process according to any one of the preceding claims, **characterized in that** the addition of X nucleotides is carried out by enzymes capable of polymerizing modified nucleotides without the presence of a matrix strand.

8. The process according to claim 7, **characterized in that** the enzyme is chosen from enzymes of the family of the telomerases, of the translation enzymes, of the template-independent RNA polymerases or enzymes of the family of the terminal deoxynucleotidyl transferases.

9. The process according to one of the claims 1 to 8, **characterized in that** the second support is covered with molecules, such as proteins, making possible a noncovalent bond between these molecules and said protective chemical group, and thus the immobilization of the fragments which have been added to during the second attaching stage.

10. The process according to any one of the preceding claims, **characterized in that** the stage of detaching said fragments comprising n + Xi nucleotides from said second support is carried out by modification of the conditions of the reaction medium, such as modifications of the pH, or of the temperature, or under the action of electromagnetic radiation if said second support is a magnetic support.

11. The process according to any one of the preceding claims, **characterized in that** the enzymatic addition is carried out in the 5' toward 3' direction.

12. The process according to any one of preceding claims, **characterized in that** it comprises c) an amplification phase, preferably enzymatic amplification, such as by PCR, of the fragments having a correct sequence comprising n+Xi nucleotides, comprising the following successive stages:
- A stage of addition of the reagents necessary for the amplification,
- A stage of multiplying by a multiplication factor Yi of the fragments comprising n+Xi nucleotides, i being the cycle number, it being possible for Y to be between 1 and 4.10¹⁰, preferably between 1 and 1.10⁹.

13. The use of the process according to any one of the preceding claims for the production of genes, or of synthetic nucleic acid sequences, of DNA or of RNA.

14. A kit for the implementation of the process according to any one of the claims 1 to 12, comprising:
- a reaction medium including nucleic acid fragments comprising n nucleotides
- an enzymatic reagent for addition of nucleotides
- nucleotides or combinations of nucleotides capable of being added by said enzymatic addition reagent, said nucleotides comprising at the 3' end a protective chemical group,
- washing (13, 14) and/or buffer solutions for the purification phase
- an amplification reaction medium including: an enzymatic reagent for amplification of nucleic acids, and natural nucleotides capable of being used by the enzymatic amplification reagent
- a first support having an attaching surface making possible a bond with 5' end of the nucleic acid fragments
- a second support having an attaching surface making possible a non-covalent bond with a protective group of the 3' end of the nucleic acid fragments
- Instructions for use.

15. The kit according to claim 14, further comprising:
- buffer media favorable to the attaching of the nucleic acid fragments to the first and/or the second attaching support, and/or
- buffer media favorable to the detaching of the nucleic acid fragments from the first and/or second attaching support.
